(19) Europäisches Patentamt
European Patent Office
Office européen des brevets

(11) Veröffentlichungsnummer: **0 211 264**
**A2**

(12) # EUROPÄISCHE PATENTANMELDUNG

(21) Anmeldenummer: **86109410.0**

(51) Int. Cl.4: **C07D 301/14** , C07D 303/04

(22) Anmeldetag: **10.07.86**

(30) Priorität: **05.08.85 DE 3528005**

(43) Veröffentlichungstag der Anmeldung:
**25.02.87 Patentblatt 87/09**

(84) Benannte Vertragsstaaten:
**AT CH DE FR GB IT LI NL SE**

(71) Anmelder: **Degussa Aktiengesellschaft**
**Weissfrauenstrasse 9**
**D-6000 Frankfurt am Main 1(DE)**

(72) Erfinder: **Grund, Andreas, Dr.**
**Rilkeweg 15**
**D-6100 Darmstadt(DE)**
Erfinder: **Prescher, Günter, Dr.**
**Liesingstrasse 2**
**D-6450 Hanau 9(DE)**
Erfinder: **Boehme, Georg**
**Nordring 49**
**D-6458 Rodenbach 1(DE)**
Erfinder: **Hofen, Willi**
**Südring 54**
**D-6458 Rodenbach 1(DE)**
Erfinder: **Petsch, Heinrich**
**Tulpenstrasse 23**
**D-6450 Hanau 8(DE)**

(54) **Verfahren zur Herstellung von aliphatischen Epoxiden.**

(57) Aliphatische Epoxide der Formel

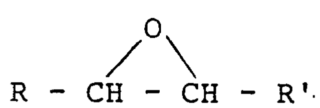

lassen sich in technisch einfacher Weise aus den entsprechenden Olefinen mit Hilfe von benzolischer Perpropionsäurelösung, die auch in ungereinigter Form mit Maximalgehalten an Wasserstoffperoxid, Wasser und Mineralsäure verwendet werden kann, herstellen.

EP 0 211 264 A2

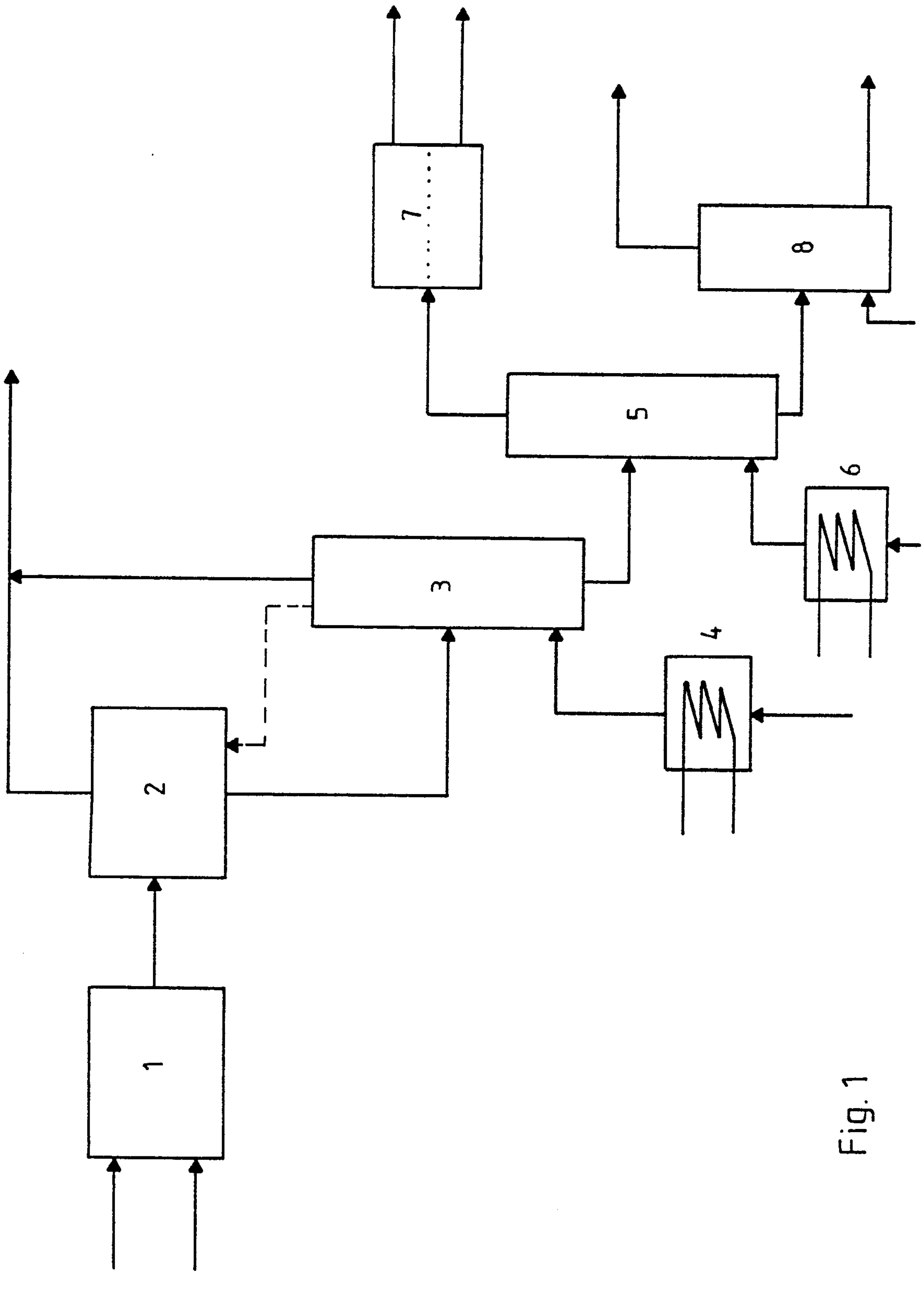


Fig. 1

# Verfahren zur Herstellung von aliphatischen Epoxiden

Die vorliegende Erfindung betrifft ein Verfahren zur Herstellung von aliphatischen Epoxiden der folgenden Formel

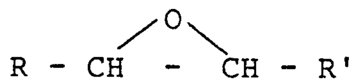

durch Epoxidation von Olefinen der Formel

$R-CH=CH-R'$,

in der R einen Alkylrest und R' einen Alkylrest oder Wasserstoff darstellen, mit einer Percarbonsäure in organischem Lösungsmittel sowie Aufarbeitung des dabei entstehenden Reaktionsgemisches.

Epoxide vorgenannter Struktur sind wertvolle Zwischenprodukte, z.B. zur Herstellung von Diolen, als Bestandteil von oberflächenaktiven Stoffen, zur Synthese einer großen Zahl organischer Zwischenprodukte sowie für den Polymerbereich.

Es ist seit langem bekannt, Epoxide durch Umsetzung von Olefinen mit Chlor im alkalischen Medium und nachfolgender Behandlung mit Basen herzustellen (Ullmann's Enzyklopädie der technischen Chemie, 3. Auflage, Band 10, Seite 565). Der wesentliche Nachteil dieses Verfahrens sind die erheblichen Mengen umweltbelastender Abwässer, die bei diesem Prozess zwangsweise anfallen.

Es ist ferner bekannt, dass Ethylen mit guten Ausbeuten in der Gasphase mit molekularem Sauerstoff an einem silberhaltigen Katalysator epoxidiert werden kann. Wegen seiner mangelnden Selektivität ist jedoch dieses Verfahren für andere Olefine ungeeignet.

Weiterhin lassen sich Olefine durch Umsetzung mit Hydroperoxiden, die aus Kohlenwasserstoffen, wie z.B. Isobutan oder Ethylbenzol, durch Oxidation mit Luft erhältlich sind (US-PS 3,351,635), in Gegenwart eines Katalysators, der Vanadin-, Molybdän-oder Wolframverbindungen enthält, in entsprechende Epoxide überführen. Diese Methode hat den gewichtigen Nachteil, dass neben der erforderlichen Abtrennung des Katalysatorsystems der aus dem Hydroperoxid als Koppelprodukt in äquimolarem Maßstab anfallende Alkohol, sofern er nicht wirtschaftlich verwertbar ist, nur unter erheblichem technischem Aufwand in das Hydroperoxid rückgeführt werden kann.

Durch Anwendung der "Prileschajew-Reaktion " (N. Prileschajew, Ber. dtsch.chem.Ges. 42, 4811 - (1909)) können die zuvor erwähnten Nachteile teilweise vermieden bzw. vermindert werden

Diese Reaktion beinhaltet im wesentlichen die Umsetzung eines Olefins mit einer organischen Percarbonsäure. Allerdings führt auch hierbei z.B. die Verwendung von Perameisen säure, die darüber hinaus in höheren Konzentrationen detonationsfähige Gemische ausbildet, zu erheblichen Abwassermengen, die einer gewissenhaften Entsorgung bedürfen. Auch ein Einsatz von Peressigsäure in wäßrigem Medium führt zu grossen Mengen verdünnter Essigsäure, die auf wirtschaftliche Weise nicht aufkonzentriert und rückgeführt werden können. Falls die Peressigsäure, wie häufig notwendig wegen der Produktstabilität, während des Prozesses mit Alkalicarbonatlösung abgepuffert und/oder nach der Reaktion mit Alkalihydroxidlösung neutralisiert wird, fallen stark salzhaltige, die Umwelt belastende Abwässer an.

Vorteile sollte daher eine Verwendung organischer Percarbonsäuren in einem organischen Lösungsmittel bieten.

So ist es bekannt, aliphatische 1,2 -Epoxide im Bereich $C_9$ -$C_{20}$ durch Umsetzung der ihnen zugrundeliegenden Olefine mit einer Lösung einer 3 bis 4 Kohlenstoffatome ethaltenden organischen Persäure herzustellen, wobei Perbenzoesäure in Benzol bevorzugt wird. (DE-OS 31 01 037 und EP-OS 056932).

Da aber nach diesem Verfahren gemäß Anspruch 1 ein Überschuss an zu epoxidierendem Olefin von 50 % bis 500 % als notwendig erachtet wird, kann dieses Verfahren nicht wirtschaftlich sein, da zum einen relativ grosse Reaktorvolumina nötig sind, und zum anderen das überschüssige Olefin aufwendig aus dem Reaktionsgemisch abgetrennt und vor einer eventuellen Rückführung in den Epoxidationsprozess gereinigt werden muss.

Im allgemeinen vertritt die Fachwelt über Epoxidation mit Persäuren die Meinung, dass die derartig mit Persäuren erhaltenen Reaktionsgemische auf Grund ihres Gehalts an Wasser und Säure, z.B. Essigsäure, sehr leicht mit den gebildeten Epoxiden unter Bildung von Nebenprodukten, wie Glykolen, Glykolmono-und diester reagieren, siehe DE-AS 15 43 032. Epoxidationsverfahren, die z.B. Perameisen-bis Perpropionsäure verwendeten, galten daher als besonders schwierig durchführbar in saurem Milieu, da hierdurch Aufspaltung des Oxiranringes eintrat, siehe DE-PS 29 16 834.

In diesem Zusammenhang ist es auch zu verstehen, dass in der DE-OS 31 01 037 und EP-OS 056932, die die Herstellung von n-Alkyloxiranen mittels Perpropionsäure beschreiben, im Anspruch 1 eine Percarbonsäurelösung mit einem Mineralsäuregehalt kleiner 50 ppm beansprucht wird. Laut Beschreibung im Text soll der Mineralsäuregehalt bevorzugt sogar kleiner 10 ppm betragen.

Nach dem genannten Stand der Technik war daher nicht zu erwarten, dass sich aliphatische Olefine mit einer benzolischen Perpropionsäure, die bis zu 1,5 Gewichtsprozent Wasserstoffperoxid, 1,5 Gewichtsprozent Wasser und 800 ppm Mineralsäure enthält, ohne wesentliche Bildung von Nebenprodukten in ihre Epoxide überführen lassen.

Aufgabe der Erfindung ist es, aliphatische Epoxide mit Hilfe von Perpropionsäure in guten Ausbeuten und unter Vermeiden von störender Nebenproduktbildung herzustellen.

Es wurde nun gefunden, dass sich diese Aufgabe lösen lässt, wenn man ein Olefin der Formel

$$R\text{-}CH = CH\text{-}R'$$

in der R einen Alkylrest und R' einen Alkylrest oder Wasserstoff darstellen, mit einer Lösung von Perpropionsäure in Benzol im Molverhältnis 1 : 1 bis 1 : 1,3, vorzugsweise 1 : 1,03 bis 1 : 1,10, (Olefin zu Perpropionsäure) bei einer Temperatur von 10 bis 100 °C umsetzt.

R und R' können Alkylreste mit zu 28 C-Atomen sein, wobei R und R' gleich oder größer als 8 ist; bevorzugt ist R = $C_8$ -$C_{28}$ mit R' = H, das heißt $\alpha$-Olefine. Ebenso können auch $\alpha$, $\omega$ -Diolefine verwendet werden, wie z.B. 1,9-Dekadien. Es ist auch möglich, Olefinschnitte z.B. im Bereich von $C_{20}$ bis $C_{30}$, bevorzugt von $C_{20}$ bis $C_{26}$, einzusetzen.

Neu und besonders vorteilhaft nach dem erfindungsgemäßen Verfahren ist es, daß beide Reaktanten in equimolaren Mengen bzw. einem nur geringen Überschuß an Persäure verwendet werden können.

Perpropionsäure kann z.B. gemäß dem in DE-PS 25 19 289 beschriebenen Verfahren hergestellt werden, indem man wäßriges Wasserstoffperoxid mit Propionsäure in Gegenwart von Schwefelsäure umsetzt und anschließend die entstandene Perpropionsäure mit Benzol aus dem Reaktionsgemisch extrahiert. Die auf diese Weise erhaltene Perpropionsäure in benzolischer Lösung kann noch weiter gereinigt werden, um den Restgehalt an Schwefelsäure, Wasser und Wasserstoffperoxid zu verringern, siehe z.B. DE-PS 25 19 290. Bevorzugt ist aber eine Perpropionsäurelösung, die keiner weiteren Reinigung bedarf; mit anderen Worten: der Rohextrakt aus der Perpropionsäureherstellung kann als solcher direkt eingesetzt werden. Dies führt zu einem erheblich verringerten technischen Aufwand.

Es kann daher eine Perpropionsäurelösung in Benzol verwendet werden, die bis 1,5 Gewichtsprozent Wasserstoffperoxid, 1,5 Gewichtsprozent Wasser und bis zu 800 ppm Mineralsäure enthält.

Nach dem erfindungsgemäßen Verfahren werden die bei Raumbzw. Reaktionstemperatur flüssig vorliegenden Olefine vorzugsweise als solche, oder aber auch verdünnt in einem geeigneten Lösungsmittel, eingesetzt, wobei man die Konzen tration in einem weiten Bereich frei wählt.

Die unter vorgenannten Bedingungen fest vorliegenden Olefine können als Schmelze oder in einem geeigneten Solvens, vorzugsweise Benzol, gelöst eingesetzt werden, wobei die Konzentration ebenfalls in einem weiten Bereich frei wählbar ist. Ausser Benzol eignen sich auch Toluol, halogenierte Kohlenwasserstoffe, wie Methylenchlorid, Chloroform, Tetrachlorkohlenstoff; besonders bevorzugt ist Benzol.

Die Umsetzung findet bevorzugt bei Temperaturen von 20 bis 70 °C statt. Das erfindungsgemässe Verfahren lässt sich unter verschiedenen Drücken durchführen; im allgemeinen wird unter Normaldruck gearbeitet, das Verfahren lässt sich aber auch bei Über-oder Unterdruck durchführen.

Die Umsetzung kann sowohl diskontinuierlich oder kontinuierlich in für diese Art der Reaktion geeigneten Reaktoren, wie Rührkesseln, Rührkesselkaskaden, Röhren-oder Schlaufenreaktoren, erfolgen, wobei die Reaktionswärme auf beliebige Weise, z.B. Siedekühlung oder innen-bzw. außenliegende Kühleinrichtungen, abgeführt wird.

Geeignete Werkstoffe für die Reaktionsapparate zur Durchführung des erfindungsgemässen Verfahrens sind z.B. Glas, Edelstahl oder emailliertes Material.

Die Perpropionsäure wird mit dem Olefin bzw. dessen Lösung in einem geeigneten Lösungsmittel auf beliebige Art zusammengebracht. So kann man beide Reaktionsteilnehmer zusammen oder nacheinander in beliebiger Reihenfolge in den Reaktor einbringen. Bei diskontinuierlicher Arbeitsweise wird vorzugsweise das Olefin vorgelegt und die Persäure unter Kontrolle der Reaktionstemperatur zudosiert. Es kann aber ebenso umgekehrt verfahren werden, d.h., man legt die Persäure vor und dosiert das Olefin unter Kontrolle der Reaktions temperatur zu. Erfolgt die Umsetzung kontinuierlich, so können beide Reaktanten getrennt oder gemeinsam dem Reaktor zugeführt werden. Bei Verwendung mehrerer hintereinandergeschalteter Reaktoren, wie z.B. einer Rührkesselkaskade oder einer Folge von Rührkesseln mit einem Rohrreaktor als Nachreaktor kann man sowohl Persäure-als auch Olefinzugabe auf mehrere Reaktoren verteilen.

Geeignete Lösungsmittel sind neben Benzol Toluol, Chlorbenzol, Halogenierte Kohlenwasserstoffe, wie Methylenchlorid, Chloroform, Tetrachlorkohlenstoff für Olefin, besonders bevorzugt wird Benzol.

Nach dem erfindungsgemässen Verfahren ist eine kontinuierliche Arbeitsweise besonders vorteilhaft. Gemäss dieser wird das aliphatische Olefin mit einer Lösung von Perpropionsäure in Benzol im Molverhältnis 1 : 1 bis 1 : 1,3 bei den genannten Temperaturen von 10 bis 100 °C in ein Reaktionssystem eingespeist, das aus einer Folge von 1 -4 ideal durchmischten Reaktoren und einem Nachreaktor besteht, wobei man die Verweilzeit so einstellt, dass der Umsatz, bezogen auf eingesetzte olefinische Doppelbindung, nach dem oder den ideal durchmischten Reaktor(en) mindestens 80 Molprozent und nach dem Nachreaktor mindestens 95, bevorzugt über 98 Molprozent, beträgt. Anschliessend wird das den Nachreaktor verlassende Reaktionsgemisch in einer Kombination von Destillations-und Desorptionsschritten von Benzol, Propionsäure, unumgesetzter Perpropionsäure sowie sonstigen flüchtigen Bestandteilen befreit. Diese Auftrennung des Reaktionsgemisches kann, da das gebildete Epoxid die Komponente mit dem höchsten Siedepunkt des Gemisches darstellt, nach einer der folgenden Varianten durchgeführt werden.

Variante 1 (diskontinuierlich)

Gemäss dieser werden die einzelnen Bestandteile des Reak tionsgemisches in der Reihenfolge ihrer Siedepunkte einzeln oder als Gemische destillativ oder destillativ und desorptiv entfernt. Hierbei gehen Fraktionen von Benzol, Resten Perpropionsäure, Propionsäure und sonstige leicht flüchtige Bestandteile über. Als Sumpf verbleibt das Epoxid. Das abgetrennte Benzol sowie die Propionsäure können gegebenenfalls nach weiteren Reinigungsschritten in die Persäureherstellung rückgeführt werden.

Variante 2 (kontinuierlich, Abbildung 1)

Nach dieser kontinuierlich durchzuführenden Variante werden zunächst, nachdem das Reaktionsgemisch die Reaktionseinheit 1 verlassen hat, Benzol, Propionsäure und nicht umgesetzte Perpropionsäure größtenteils in der ein-oder mehrstufigen Destillationseinheit 2 entfernt. Diese besteht aus geeigneten Apparaten, wie Dünnschicht-, Fallfilm-oder Umlaufverdampfern. Es ist vorteilhaft, unter vermindertem Druck von 0.5 bis 600, vorzugsweise 10 bis 300 mbar, zu destillieren. - (Temperatur des Heizmediums 50 bis 150 °C). Die mittleren Verweilzeiten, bezogen auf die einzelnen Stufen der Verdampfung, liegen bei maximal 10 min, bevorzugt werden Verweilzeiten von maximal 5 min.

Anschliessend wird nach dem erfindungsgemässen Verfahren die im Rohprodukt verbliebene Menge Propionsäure in der Desorptionseinheit 3 mit Benzoldampf, der im Verdampfer 4 generiert wird, desorptiv entfernt bei Temperaturen des Heizmediums von 50 bis 150 °C und einem Druck von 0,5 bis 600, vorzugsweise 10 bis 300 mbar. Nach diesem Schritt werden aus dem Epoxid die verbliebenen Spuren Benzol mit Wasserdampf aus dem Verdampfer 6 in der Desorptionseinheit 5 und/oder Stickstoff bzw. sonstigen Inertgasen in der Desorptionseinheit 8 desorbiert. Es ist besonders bevorzugt, zunächst mit Wasserdampf und anschliessend mit Inertgas zu desorbieren. Das aus der Desorptionseinheit 5 stammende Kondensat trennt sich im Phasentrenner 7 in Wasser, welches dem Verdampfer 6, gegebenenfalls nach Ergänzung, zurückgeführt werden kann; die organische Phase, die vorwiegend Benzol und Propionsäure enthält, wird gegebenenfalls nach weiterer Aufarbeitung, der Perpropionsäureherstellung oder Epoxidation zurückgeführt. Ebenfalls werden die aus den Destillations-bzw. Desorptionseinheiten 2 und 3 stammenden Kondensatströme, die im wesentlichen aus Benzol, nicht umgesetzter Perpropion-und Propionsäure bestehen, nach weiterer Auftrennung -siehe Abbildung 3 -deren Beschreibung später erfolgt, in die Persäureherstellung bzw. Epoxidation rückgeführt.

Variante 3 (kontinuierlich, Abbildung 2)

Gemäss der kontinuierlich anzuwendenden Variante 3 werden, wie bei Variante 2, Benzol, unumgesetzte Perpropionsäure und Propionsäure in der ein-oder mehrstufigen Destillationseinheit 2 enfernt. Anschliessend wird in der Desorptionseinheit 3 restliche Propionsäure mit Benzoldampf desorbiert. Zur Entfernung von verbliebenen Spuren an Propionsäure wird nun das Rohepoxid, gegebenenfalls nach Verdünnen bzw. Lösen mit einem mit Wasser nicht mischbaren Lösungsmittel, vorzugsweise Benzol, mit wäßrigen Alkalien in der Extraktion 9 und anschliessend mit Wasser in der ein-oder mehrstufigen Extraktionseinheit 10 gewaschen.

Geeignete Apparate für diese Schritte sind Extraktionskolonnen verschiedener Bauart oder auch Mixer-Settler-Einheiten, deren Betriebsweise und Auslegung dem Fachmann wohlbekannt sind. Als wäßrige Alkalilösungen können Lösungen von z.B. NaOH, KOH, $Na_2CO_3$, $K_2CO_3$, $NaHCO_3$, $KHCO_3$, $NH_3$ usw. eingesetzt werden, wobei deren Konzentration in einem weiten Bereich frei wählbar ist. Bevorzugt wird eine NaOH-Lösung mit einer Konzentration zwischen 0,01 bis 5 Gewichtsprozent, besonders bevorzugt 0,01 bis 1,0 Gewichtsprozent.

Bei Einsatz von Mixer-Settler-Einheiten für die Wäsche mit Wasser kann das Wasser im Gegenstrom geführt werden, es kann aber auch jede Einheit mit Frischwasser betrieben werden. Vorteilhaft wird ein Teil des Abwassers aus den Mixer-Settler-Einheiten zum Ansetzen der Alkali-Lösung verwendet. Die Alkali-und Wasserwäsche kann in einem Temperaturbereich von 10 bis 90 °C betrieben werden, bevorzugt werden Temperaturen von 30 bis 70 °C. In der Alkali-Wäsche beträgt das Gewichtsverhältnis von durchgesetztem Epoxid zu Alkalilösung 1 : 1 bis 100 : 1, in der Wasserwäsche liegt das Verhältnis von Epoxiddurchsatz zu Wasserdurchsatz bei 1 : 1 bis 100 : 1.

Im Anschluss an die Wasserwäsche erfolgt die weitere Aufarbeitung durch Desorption mit Wasserdampf und/oder Inertgas, wie bei Variante 2 beschrieben.

Bei allen Varianten durch Kombination von Destillations-und Desorptionsschritten fallen Kondensate an, die überwiegend aus Benzol, unumgesetzter Perpropionsäure, Propionsäure und sonstigen Leichtsiedern bestehen. Sie werden nach dem erfindungsgemässen Verfahren in eine aus einer oder mehreren Kolonnen bestehende Destillationseinheit 11 (Zeichnung 3) überführt. Diese liefert als Kopfprodukt Benzol und gegebenenfalls weitere Leichtsieder. Ersteres wird gegebenenfalls nach weiterer Destillation in 12 in das Herstellverfahren der Perpropionsäure zurückgeführt. Im Sumpf der Destillationseinheit 11 fällt ein Gemisch aus Propionsäure, Perpropionsäure und Benzol mit einem Benzolanteil von 5 bis 35 Gewichtsprozent, bezogen auf die Sumpfmischung, an. Dieses Gemisch wird einer weiteren Destillationseinheit 13 zugeführt, in der man die Gesamtmenge des zugeführten Benzols und der Perpropionsäure mit Anteilen Propionsäure über Kopf abzieht und dabei eine Konzentration von Perpropionsäure im Destillat von 25 Gewichtsprozent nicht überschrei tet und dieses Kopfprodukt in das Herstellverfahren der Perpropionsäure oder in die Umsetzung des Olefins mit Perpropionsäure zurückführt. Als Sumpfprodukt in Kolonne 13 fällt Propionsäure an, die nach weiterer Aufarbeitung wie Reindestillation in die Herstellung der Perpropionsäure, gegebenenfalls nach Ergänzung, zurückgeführt wird. Besonders vorteilhaft ist es, die in 13 anfallende Propionsäure dampfförmig oberhalb des Sumpfes abzuziehen und zu kondensieren, da hierdurch der weitere Reinigungsschritt entfällt.

Erfindungsgemäss werden alle destillativen bzw. desorptiven Aufarbeitungsschritte vorzugsweise unter vermindertem Druck, z.B. 0,5 bis 600 mbar, durchgeführt. Kolonnen, in denen Benzol oder Propionsäure als Kopfprodukt anfällt, können ebenso bei Normaldruck betrieben werden.

Bei der Aufarbeitung der Reaktionsgemische nach allen Varianten fällt das Epoxid als hochsiedende Komponente, nämlich als Sumpfprodukt, an. Je nach Anforderungen des Einsatzzweckes bzw. der Weiterverarbeitung kann das Epoxid direkt oder aber nach einer weiteren Feindestillation, die vorzugsweise im Vakuum stattfindet, eingesetzt werden.

Das erfindungsgemässe Verfahren bietet eine Reihe von überraschenden Vorteilen.

Mit Hilfe der sog. Prileschajew-Reaktion ist es nach diesem Verfahren möglich, die vorgenannten Epoxide im technischen Maßstab auf gefahrlose Weise in hoher Ausbeute herzustellen. Das auf diese Weise erhaltene Produkt zeichnet sich durch ausserordentliche Reinheit, hohen Epoxidgehalt und helle Farbe aus.

Das beschriebene Verfahren ist wirtschaftlich, da die Reaktanten annähernd äquivalent eingesetzt und alle Hilfsmedien rückgeführt werden. Das Verfahren ist besonders umweltfreund lich, da aus dem Oxidationsmittel lediglich Wasser als Abfall entsteht; darüber hinaus fallen nur geringe Mengen an sonstigen Abwässern, Leichtsiedern und Destillationsrückständen an, die unproblematisch und gefahrlos entsorgt werden können.

Erfindungsgemäss sind nur kurze Reaktionszeiten nötig, was die technische Durchführung besonders wirtschaftlich gestaltet.

Es war überraschend und nicht vorhersehbar, dass die Umsetzung der vorgenannten Olefine mit einer rohen Perpropionsäure, die noch Mineralsäure, Wasser und Wasserstoffperoxid in den vorgenannten Konzentrationen enthält, bei weitestgehender Unterdrückung von Neben-und Folgereaktionen durchführbar ist. Weiterhin war nicht vorhersehbar, dass die dabei anfallenden Reaktionsgemische erfindungsgemäss destillativ oder destillativ und desorptiv aufarbeitbar sind, ohne daß sich dadurch der Epoxidgehalt des Produktes merklich verringert.

Beispiel 1 (diskontinuierlich)

Zu 840 g (6,0 mol) 1-Decen wurden unter Rühren und Kühlen auf 50 °C innerhalb 1 h 2700 g (6,6 mol) Perpropionsäure (22 Gew.-%) in Benzol gegeben. Anschliessend rührte man 2 h bei 60 °C nach. Der Umsatz an Olefin betrug dann 98,4 %. Die erhaltene klare, schwach gelbe Lösung wurde innerhalb 2,5 h bei 80°C und einem Druck von 100 mbar über einen Dünnschichtverdampfer gegeben, wobei gleichzeitig im Gegenstrom ca. 347 g/h Benzoldampf geführt wurden. Das so erhaltene Rohepoxid wurde nun bei 80 °C/30 mbar über den Dünnschichtverdampfer gegeben; im Gegenstrom wurde ein schwacher Stickstoffstrom geführt.

Als Sumpf erhielt man 902 g Decenoxid mit einem Epoxidgehalt von 96,5 %.

Beispiel 2 (diskontinuierlich)

Zu 1008 g (4,0 mol) 1-Octadecen wurden unter Rühren und Kühlen auf 60 °C innerhalb 1,5 h 1718 g (4,2 mol) Perpropionsäure (22 Gew.-%) in Benzol gegeben. Anschliessend rührte man 3 h bei 70 °C nach. Der Umsatz an Olefin betrug dann 98,4 %. Die erhaltene klare, schwach gelbe Lösung wurde innerhalb 2,5 h bei 90 °C und einem Druck von 100 mbar über einen Dünnschichtverdampfer gegeben, wobei gleichzeitig im Gegenstrom ca. 230 g/h Benzoldampf geführt wurden. Das so erhaltene Rohepoxid wurde nun bei 90 °C/20 mbar über den Dünnschichtverdampfer gegeben; im Gegenstrom wurde ein schwacher Stickstoffstrom geführt.

Als Sumpf erhielt man 1057 g Octadecenoxid mit einem Epoxidgehalt von 95,5 %.

Beispiel 3 (diskontinuierlich)

Zu 361 g eines $C_{24}$-$C_{28}$-α-Olefingemisches - (mittlere Molmasse 360) wurden unter Rühren und Kühlen auf 55 °C innerhalb 45 min 430 g (1,05 mol) Perpropionsäure (22 Gew.-%) in Benzol gegeben. Anschliessend rührte man 4 h bei 70 °C nach. Der Umsatz an Olefin betrug dann 97,8 %. Die erhaltene klare, schwach gelbe Lösung wurde innerhalb 60 min bei 90 °C und einem Druck von 100 mbar über einen Dünnschichtverdampfer gegeben, wobei gleichzeitig im Gegenstrom ca. 315 g Benzoldampf geführt wurden. Das so erhaltene Rohepoxid wurde nun bei 90 °C/20 mbar über den Dünnschichtverdampfer gegeben; im Gegenstrom wurde ein schwacher Stickstoffstrom geführt.

Als Sumpf erhielt man 369 g festes Epoxidgemisch, welches laut $^1$H -NMR-Spektrum keine Doppelbindungen mehr aufwies.

Beispiel 4 (kontinuierlich)

In den ersten Rührkessel einer Reaktionseinheit, bestehend aus zwei Rührkesseln vom Volumen jeweils 1500 ml sowie einem als Rohrreaktor ausgebildeten Nachreaktor, der ein Volumen von 790 ml hat, wurden stündlich 1,43 mol Perpropionsäure in Benzol (ca. 22 Gew.-%) und 1,3 mol 1-Tetradecen eingespeist, was einem Molverhältnis Persäuren zu Olefin von 1,1 : 1 entspricht. Die Reaktionstemperatur betrug im ersten Reaktor 50 °C, im zweiten Reaktor 51 °C und im Nachreaktor 70 °C. Die Umsätze an Olefin betrugen nach der Rührkesselkaskade 92,3 %, nach dem Rohrreaktor 98,4 %. Gemäß Aufarbeitungsvariante 2 wurden zunächst in einem Sambay-Verdampfer mit der Fläche von 0.065 m² bei einer Temperatur von 83 °C und einem Druck von 100 mbar Benzol, Perpropionsäure und Propionsäure abgetrennt. Restliche Propionsäure wurde in einem zweiten Verdampfer gleichen Typs und gleicher Fläche bei 86 °C und 100 mbar bei einem Durchsatz von 405 g/h Benzoldampf desorbiert. Die Brüden des zweiten Verdampfers wurden vollständig im Gegenstrom zum Produktstrom in den ersten Verdampfer eingeführt. Nachfolgend wurde das Epoxid in zwei Desorptionseinheiten, bestehend aus jeweils einem Sambay-Verdampfer (Fläche 0,065 m²), bei 40 mbar mit 31 g/h Wasserdampf und bei 30 mbar mit 14 g/h Stickstoff bei Temperaturen von 95 °C behandelt. Als Produkt fielen stündlich 272,0 g Epoxid mit 96,4 % Epoxidgehalt an.

Beispiel 5 (kontinuierlich)

In den ersten Rührkessel einer Reaktionseinheit, bestehend aus zwei Rührkesseln vom Volumen jeweils 1500 ml sowie einem als Rohrreaktor ausgebildeten Nachreaktor, der ein Volumen von 790 ml hat, wurden stündlich 1,55 mol Perpropionsäure in Benzol (ca. 22 Gew.-%) und 1,48 mol 1-Octadecen eingespeist, was einem Molverhältnis Persäure zu Olefin, von 1,05 : 1 entspricht. Die Reaktionstemperatur betrug im ersten Reaktor 60 °C, im zweiten Reaktor 60 °C und im Nachreaktor 70 °C. Die Umsätze an Olefin betrugen nach der Rührkesselkaskade 91,9 %, nach dem Rohrreaktor 98,6 %. Gemäß Aufarbeitungsvariante 2 wurden zunächst in einem Sambay-Verdampfer mit der Fläche von 0,065 $m^2$ bei einer Temperatur von 80 °C und einem Druck von 100 mbar Benzol, Perpropionsäure und Propionsäure abgetrennt. Restliche Propionsäure wurde in einen zweiten Verdampfer gleichen Typs und gleicher Fläche bei 80 °C und 100 mbar bei einem Durchsatz von 299 g/h Benzoldampf desorbiert. Die Brüden des zweiten Verdampfers wurden nicht in den ersten Verdampfer geführt. Nachfolgend wurde das Epoxid in zwei Desorptionseinheiten, bestehend aus jeweils einem Sambay-Verdampfer (Fläche 0,065 $m^2$), bei 40 mbar mit 30 g/h Wasserdampf und bei 30 mbar mit 14 g/h Stickstoff bei Temperaturen von 90 °C behandelt. Als Produkt fielend stündlich 390,8g Epoxid mit 94,6 % Epoxidgehalt an.

Beispiel 6 (kontinuierlich)

In den ersten Rührkessel einer Reaktionseinheit, bestehend aus zwei Rührkesseln vom Volumen jeweils 1500 ml sowie einem als Rohrreaktor ausgebildeten Nachreaktor, der ein Volumen von 1900 ml hat, wurden stündlich 2,0 mol Perpropionsäure in Benzol (ca. 22 Gew.-%) und 1,9 mol 1-Dodecen eingespeist, was einem Molverhältnis Persäure zu Olefin von 1,05 : 1 entspricht. Die Reaktionstemperatur betrug im ersten Reaktor 50 °C, im zweiten Reaktor 50 °C und im Nachreaktor 60 °C. Die Umsätze an Olefin betrugen nach der Rührkesselkaskade 92,7 %, nach dem Rohrreaktor 98,5 %. Gemäß Aufarbeitungsvariante 3 wurden zunächst in einem Sambay-Verdampfer mit der Fläche von 0,065 $m^2$ bei einer Temperatur von 90 °C und einem Druck von 100 mbar Benzol, Perpropionsäure und Propionsäure abgetrennt. Restliche Propionsäure wurde in einem zweiten Verdampfer gleichen Typs und gleicher Fläche bei 87 °C und 100 mbar bei einem Durchsatz von 320 g/h Benzoldampf desorbiert. Das so als Sumpf erhaltene Rohepoxid wurde nun in einem Mixer-Settler-System mit 0,1 %iger Natronlauge (220 ml/h) und anschließend in einer folge von drei Mixer-Settler-Einheiten mit Wasser (jeweils 180 ml/h) gewaschen. Nachfolgend wurde das Epoxid in zwei Desorptionseinheiten, bestehend aus jeweils einem Sambay-Verdampfer (Fläche 0,065 $m^2$), bei 18 mbar mit 43 g/h Waserdampf und bei 21 mbar mit 16 g/h Stickstoff bei Temperaturen von 95 °C behandelt. Als Produkt fielen stündlich 340,1 g Epoxid mit 95,0 % Epoxidgehalt an.

In allen Versuchen wurde die Persäure nach der DE-PS 25 19 289 hergestellt und enthält 0,57 Gew.-% Wasserstoffperoxid, 0,90 Gew.-% Wasser und 620 ppm Schwefelsäure.

Beim erfindungsgemäßen Verfahren kann der Persäuregehalt der Lösungen zwischen 10 und 30 Gewichtsprozent liegen.

## Ansprüche

1.) Verfahren zur Herstellung von aliphatischen Epoxiden der folgenden Formel

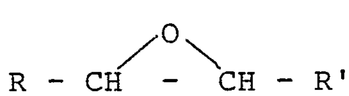

durch Epoxidierung von Olefinen der Formel

R -CH $=$ CH -R',

in der R einen Alkylrest und R' einen Alkylrest oder Wasserstoff darstellen, mit einer Percarbonsäure in organischer Lösung, dadurch gekennzeichnet, daß man ein Olefin der obengenannten Formel mit einer Lösung von Perpropionsäure in Benzol im Molverhältnis 1 : 1 bis 1 : 1,3 (Olefin zu Perpropionsäure), bevorzugt 1 : 1,03 bis 1 : 1,10, bei einer Temperatur von 10 bis 100 °C, vorzugsweise 20 bis 70 °C, umsetzt.

2.) Verfahren nach Anspruch 1, dadurch gekennzeichnet, daß man als Olefine solche nimmt, bei denen R Alkyl reste von $C_1$ -$C_{28}$ und R' Wasserstoff oder Alkylreste von $C_1$ -$C_{28}$ bedeuten, wobei die Summe von R und R' gleich oder größer als 8 ist.

3.) Verfahren nach den Ansprüchen 1 und 2, dadurch gekennzeichnet, daß R einen Alkylrest mit 8 bis 28 Kohlenstoffatomen und R' Wasserstoff darstellen.

4.) Verfahren nach den Ansprüchen 1 und 2, dadurch gekennzeichnet, daß R' = Wasserstoff und R einen endständig ungesättigten Alkylrest darstellen.

5.) Verfahren nach Anspruch 1, dadurch gekennzeichnet, daß die Perpropionsäurelösung maximal einen Gehalt von 1,5 Gewichtsprozent Wasserstoffperoxid, 1,5 Gewichtsprozent Wasser und ca. 800 ppm Mineralsäure besitzt.

6.) Kontinuierliches Verfahren nach den Ansprüchen 1 und 2, dadurch gekennzeichnet, daß man das aliphatische Olefin mit einer Lösung von Perpropionsäure in Benzol im Molverhältnis 1 : 1 bis 1 : 1,3 in ein Reaktionssystem einspeist, das aus einer Folge von 1 bis 4 ideal durchmischten Reaktoren und einem Nachreaktor besteht, die Reaktion bei einer Temperatur von 10 bis 100 °C durchführt, wobei man die Verweilzeit so einstellt, daß der Umsatz, bezogen auf eingesetzte olefinische Doppelbindung, nach dem oder den ideal durchmischten Reaktoren bei mindestens 80 Molprozent und nach dem Nachreaktor bei mindestens 95, bevorzugt über 98 Molprozent, liegt, und daß man das aus dem Nachreaktor austretende Gemisch in einer Kombination von Destillations-und Desorptionsschritten von Benzol, Propionsäure, geringen Mengen an Perpropionsäure und von anderen Leichtsiedern befreit.

7.) Verfahren nach den Ansprüchen 1 bis 5, dadurch gekennzeichnet, daß man die Destillations- und Desorptionsschritte unter vermindertem Druck von 0,5 bis 600 mbar bei Temperaturen des Heizmediums von 50 bis 150 °C und bei Verweilzeiten von maximal 10 Minuten, bevorzugt maximal 5 Minuten, in den einzelnen Schritten durchführt.

8.) Verfahren nach den Ansprüchen 1 bis 7, dadurch gekennzeichnet, daß man zunächst Benzol und Propionsäure sowie die geringen Mengen Perpropionsäure größtenteils destillativ abtrennt, worauf man die verbliebene Menge Propionsäure im Rohepoxid mit Benzoldampf weiter desorptiv entfernt und entweder hieran direkt anschließend das Benzol und Spuren von Propionsäure desorptiv mit Wasserdampf und/oder Inertgasen austreibt, oder daß man nach der Desorption mit Benzoldampf das rohe Epoxid zunächst mit wäßrigen Alkalien und anschließend mit Wasser wäscht und erst dann die Desorption mit Wasserdampf und/oder Inertgasen anschließt.

9.) Verfahren nach den Ansprüchen 1 bis 8, dadurch gekennzeichnet, daß man das aus der Kombination von Destillations-und Desorptionsschritten erhaltene Gemisch aus Benzol, Propionsäure, geringen Mengen Perpropionsäure, sowie gegebenenfalls anderen Leichtsiedern in eine aus zwei oder mehreren Destillationskolonnen bestehende Destillationsanlage führt und in dem ersten Destillationsschritt über Kopf Benzol, gegebenenfalls im Gemisch mit anderen Leichtsiedern, abzieht, das man gegebenenfalls nach destillativer Reinigung in das Herstellungsverfahren der Perpropionsäure wieder zurückführt, und daß man im Sumpf die Gesamtmenge an Perpropionsäure und Propionsäure, sowie Anteile von Benzol in Mengen von 5 bis 35 Gewichtsprozent, bezogen auf die Sumpfmischung, entimmt und diese Mischung in eine zweite Destillationsstufe führt, in der man die Gesamtmenge des darin enthaltenen Benzols und der Perpropionsäure mit Anteilen an Propionsäure über Kopf abzieht und dabei eine Konzentration von Perpropionsäure in dem Kopfprodukt von mehr als 25 Gewichtsprozent nicht überschreitet, dieses Kopfprodukt in das Herstellungsverfahren der Perpropionsäure oder in die Umsetzung der Perpropionsäure mit Olefin zurückführt, und daß man die Propionsäure als Sumpfprodukt gegebenenfalls dampfförmig oberhalb des Sumpfes abzieht und in das Herstellungsverfahren der Perpropionsäure zurückführt.

10.) Verfahren nach den Ansprüchen 1 bis 9, dadurch gekennzeichnet, daß als Ausgangsolefin ein Olefingemisch aus $C_{20}$ -$C_{30}$, bevorzugt aus $C_{20}$ -$C_{26}$, eingesetzt wird.

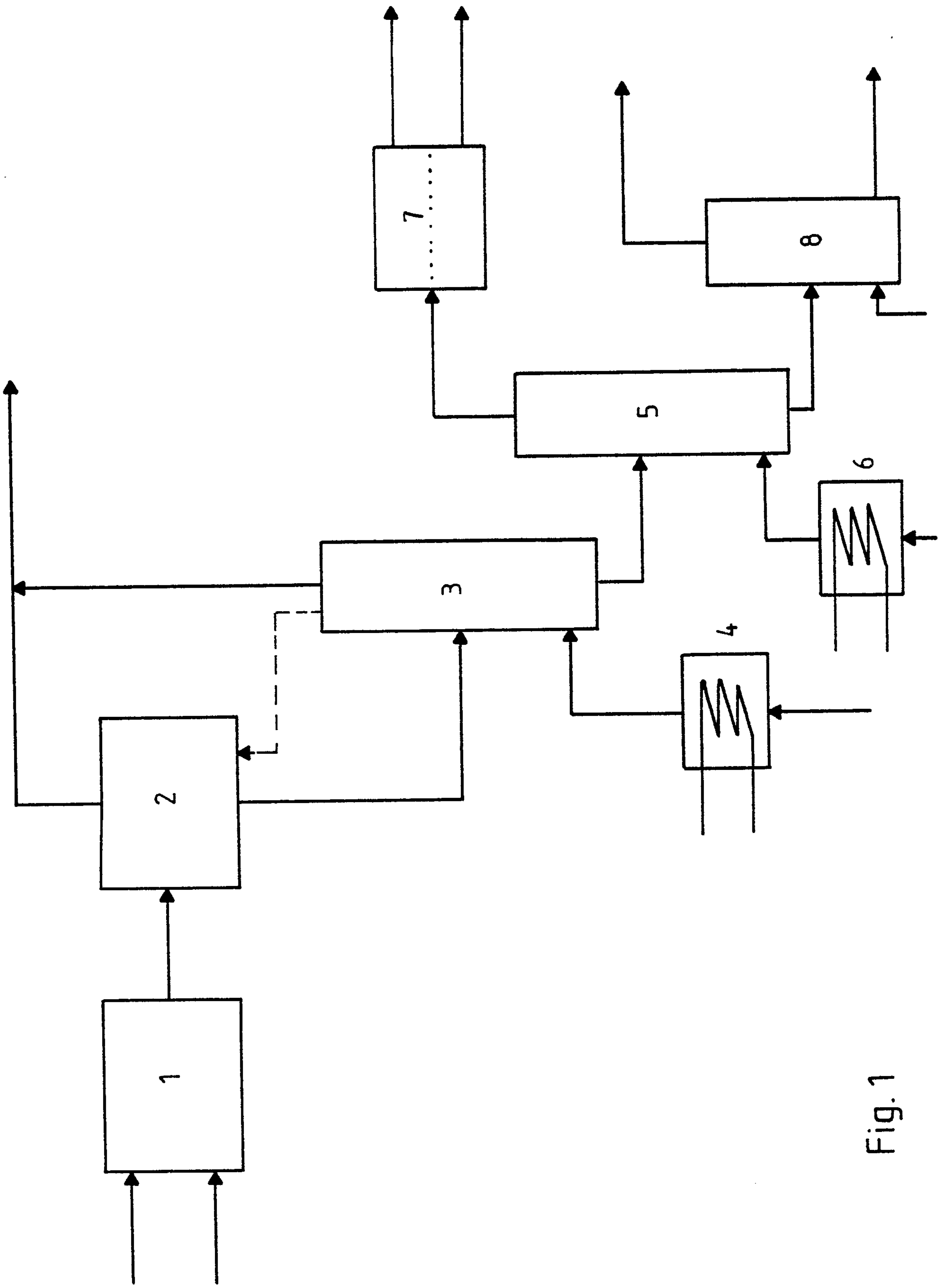

Fig. 1

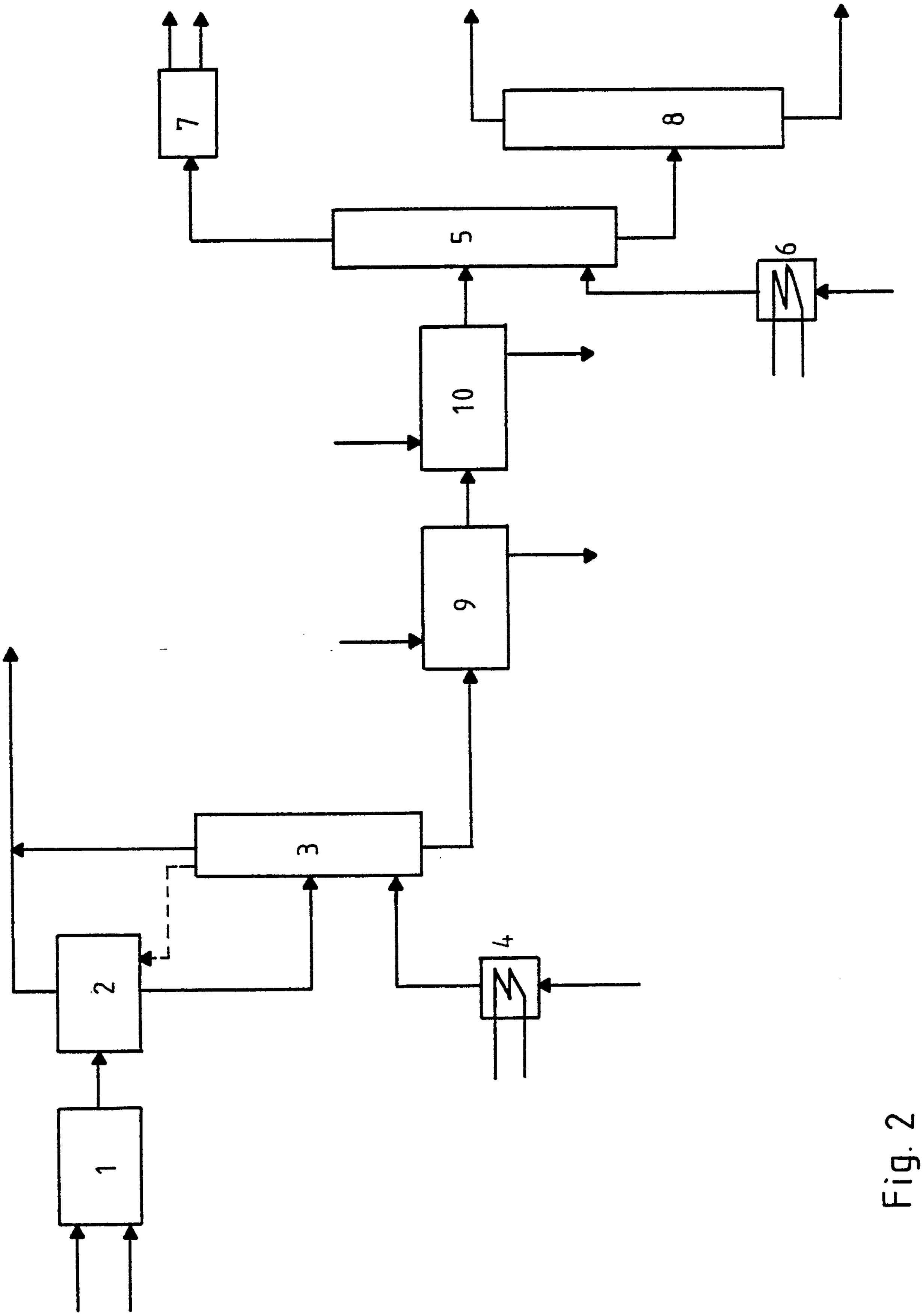


Fig. 2

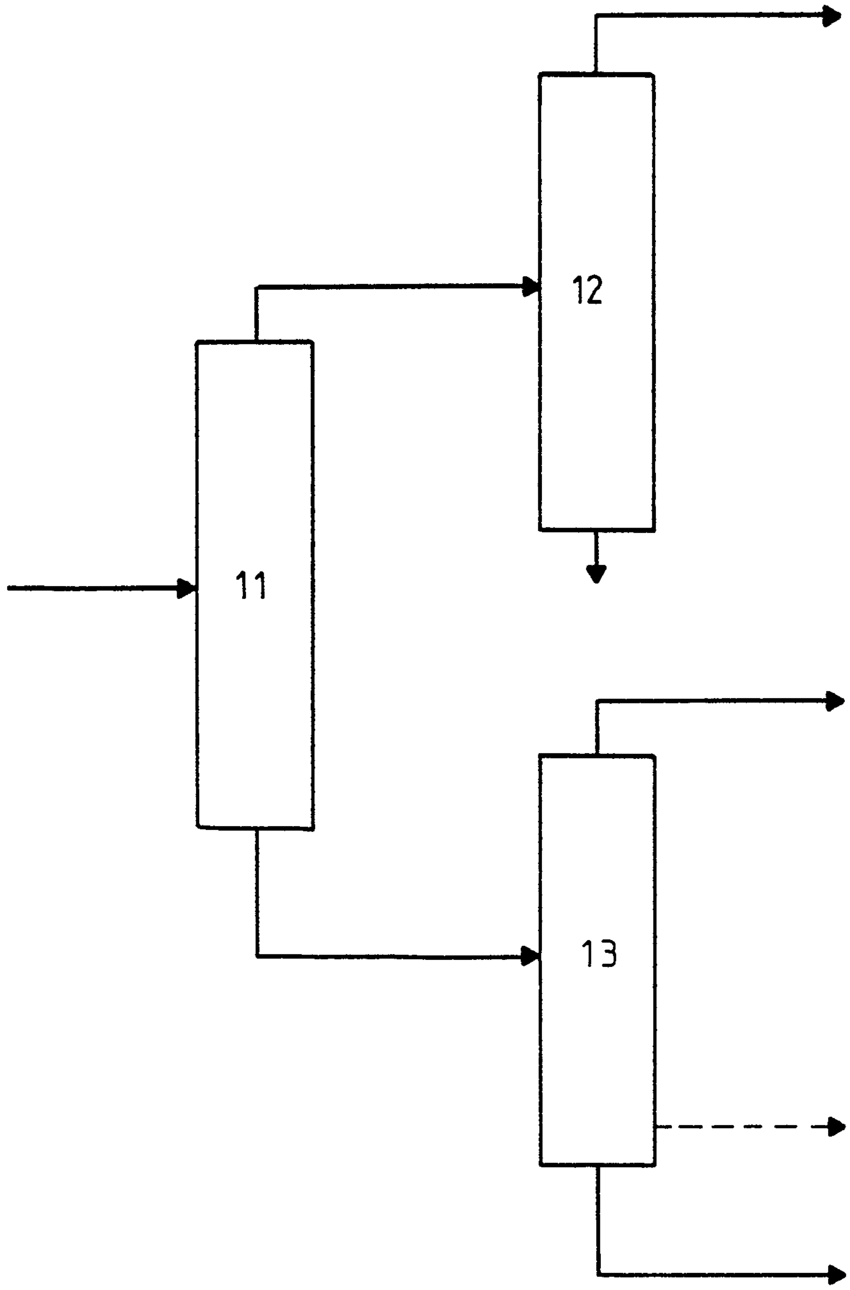


Fig. 3